(19) Europäisches Patentamt
European Patent Office
Office européen des brevets

(11) EP 2 915 536 B1

(12) **EUROPEAN PATENT SPECIFICATION**

(45) Date of publication and mention of the grant of the patent:
**21.08.2019 Bulletin 2019/34**

(21) Application number: **13850673.8**

(22) Date of filing: **18.10.2013**

(51) Int Cl.:
*A61K 31/7048* (2006.01) *A61P 35/00* (2006.01)
*A61K 36/00* (2006.01) *A61K 31/704* (2006.01)
*A61K 36/185* (2006.01)

(86) International application number:
**PCT/JP2013/078310**

(87) International publication number:
**WO 2014/069255 (08.05.2014 Gazette 2014/19)**

# (54) ANTICANCER AGENT, AND COMBINATION USE ANTICANCER AGENT

ANTIKREBSMITTEL UND KOMBINATION MIT DEM ANTIKREBSMITTEL

AGENT ANTI-CANCÉREUX, ET AGENT ANTI-CANCÉREUX DESTINÉ À ÊTRE UTILISÉ AU SEIN D'UNE POLYTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2012 JP 2012241685**
**26.03.2013 JP 2013064156**

(43) Date of publication of application:
**09.09.2015 Bulletin 2015/37**

(73) Proprietor: **Microbial Chemistry Research Foundation**
**Tokyo 141-0021 (JP)**

(72) Inventors:
- **TATSUDA, Daisuke**
**Tokyo 141-0021 (JP)**
- **MOMOSE, Isao**
**Tokyo 141-0021 (JP)**
- **KITAYAMA, Takashi**
**Nara-shi**
**Nara 631-8505 (JP)**

(74) Representative: **Graf von Stosch, Andreas et al**
**Graf von Stosch**
**Patentanwaltsgesellschaft mbH**
**Prinzregentenstraße 22**
**80538 München (DE)**

(56) References cited:
**WO-A1-2013/155175**
**CN-A- 101 152 192**
**US-A1- 2005 070 467**

- **SALEEM A ET AL: "Inhibition of cancer cell growth by crude extract and the phenolics of Terminalia chebula retz. fruit", JOURNAL OF ETHNOPHARMACOLOGY, ELSEVIER IRELAND LTD, IE, vol. 81, no. 3, 1 January 2002 (2002-01-01), pages 327-336, XP003014690, ISSN: 0378-8741, DOI: 10.1016/S0378-8741(02)00099-5**
- **KUO ET AL: "Rugosin E, an ellagitannin, inhibits MDA-MB-231 human breast cancer cell proliferation and induces apoptosis by inhibiting nuclear factor-kappaB signaling pathway", CANCER LETTERS, NEW YORK, NY, US, vol. 248, no. 2, 7 March 2007 (2007-03-07) , pages 280-291, XP005912883, ISSN: 0304-3835, DOI: 10.1016/J.CANLET.2006.08.006**
- **JANG-CHANG LEE ET AL: "Geraniin-mediated apoptosis by cleavage of focal adhesion kinase through up-regulation of Fas ligand expression in human melanoma cells", MOLECULAR NUTRITION & FOOD RESEARCH, vol. 52, no. 6, 1 June 2008 (2008-06-01), pages 655-663, XP055253063, DE ISSN: 1613-4125, DOI: 10.1002/mnfr.200700381**
- **YI ZONG-CHUN ET AL: "Tellimagrandin I enhances gap junctional communication and attenuates the tumor phenotype of human cervical carcinoma HeLa cells in vitro", CANCER LETTERS, vol. 242, no. 1, 2006, pages 77-87, XP029240452, ISSN: 0304-3835, DOI: 10.1016/J.CANLET.2005.10.044**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MIYAMOTO KENICHI ET AL.: 'Relationship between the Structures and the Antitumor Activities of Tannins' CHEMICAL & PHARMACEUTICAL BULLETIN vol. 35, no. 2, 1987, pages 814 - 822, XP055245032**
- **JOURNAL OF CLINICAL AND EXPERIMENTAL MEDICINE vol. 164, no. 5, 1993, pages 251 - 254, XP008178804**
- **MERCK MANUAL 18TH EDITION JAPANESE LANGUAGE EDITION 2007, page 1228, XP008178805**

## Description

Technical Field

The present invention relates to an anticancer agent which is geraniin and a combination use anticancer agent comprising geraniin and doxorubicin.

Background Art

Various treatments have been conducted as treatments to cancer patients, but in particular, treatments with chemotherapeutic agents have widely been conducted. Cancer cells proliferate more frequently than normal cells do, and hence conventionally a lot of drugs that suppress and stop proliferation of cells have been used as anticancer agents in clinical practice. However, they do not have sufficient antitumor effects, and do cause adverse side effects, which is problematic.

Under such circumstances, more usuful new anticancer agents have been explored, and there is a keen demand to rapidly provide such agents.

Ellagitannin is a compound known to be widely present in the plant kingdom. So far, proposed are antihepatitis B virus agents containing the ellagitannin (see, for example, PTL 1), preventive/therapeutic agents for bone diseases containing the ellagitannin (see, for example, PTL 2), and hair growing agents containing the ellagitannin (see, for example, PTL 3). However, the ellagitannin has not been known to have antitumor effects.

NPTL 1 suggests that geraniin may be a potential anticancer agent. However, it does not envisage the treatment of specific cancers or tumors such as osteosarcoma or lung cancer.

Citation List

Patent Literature

PTL 1: Japanese Patent Application Laid-Open (JP-A) No. 04-36239
PTL 2: JP-A No. 06-183958
PTL 3: JP-A No. 2004-91390

Non-Patent Literature

NPTL 1: Lee et al. Mol Nutr Food Res. 2008 Jun;52(6):655-63.

Summary of Invention

Technical Problem

The present invention aims to solve the above existing problems and achieve the following object. That is, an object of the present invention is to provide an anticancer agent and a combination use anticancer agent each having excellent anticancer effects.

Solution to Problem

Means for solving the above problem are as follows.

<1> An anticancer agent for use in preventing or treating osteosarcoma or lung cancer, the anticancer agent comprising:
geraniin.
<2> A combination use anticancer agent,
wherein an anticancer agent containing geraniin and an anticancer agent containing doxorubicin are used in combination.

Advantageous Effects of Invention

The present invention can achieve the above object and provide an anticancer agent and a combination use anticancer agent each having excellent anticancer effects.

Brief Description of Drawings

Fig. 1 is a graph indicating a change of tumor volume in mice transplanted with human osteosarcoma SJSA-1 cells in Test Example 2-1.
Fig. 2 is a graph indicating a change of tumor volume in mice transplanted with human lung cancer NCI-H460 cells in Test Example 2-1.
Fig. 3 is a graph indicating a change of tumor volume in mice transplanted with human osteosarcoma SJSA-1 cells in Test Example 2-2.
Fig. 4 is a graph indicating a change of tumor volume in mice transplanted with human osteosarcoma SJSA-1 cells in Test Example 2-3.
Fig. 5 is a graph indicating a change of tumor volume in mice transplanted with human osteosarcoma SJSA-1 cells in Test Example 3.

Description of Embodiments

(Anticancer agent)

An anticancer agent for the use of the present invention (hereinafter may be referred to as "ellagitannin-containing anticancer agent") contains at least the ellagitannin geraniin; and, if necessary, further contains other ingredients.

<Ellagitannin>

Ellagitannin is a substance that is widely present in the plant kingdom and is known to have strong antioxidative effects.
It preferably contains at least one hexahydroxydiphenoyl group (see the following "Formula I") in a molecule thereof. Ellagitannin containing at least one hexahydroxydiphenoyl group in a molecule thereof is hydrolyzed to produce ellagic acid.

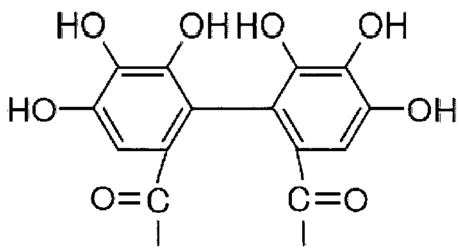

Formula I

Specific examples disclosed herein of the ellagitannin include geraniin, casuarictin, eugeniin, tellimagrandin I, 1,3-di-O-galloyl-4,6-O-hexahydroxydiphenoyl glucose, strictinin, sanguiin H-1, sanguiin H-4, 2,3-hexahydroxydiphenoyl glucose, chebulagic acid, pedunculagin, isoterchebin, granatin A, granatin B, chebulinic acid, casuarinin, nupharin C, nupharin D, nupharin F, and sanguiin H-11.
Among them, geraniin, casuarictin, eugeniin, tellimagrandin I, 1,3-di-O-galloyl-4,6-O-hexahydroxydiphenoyl glucose, and strictinin are preferable, and geraniin and eugeniin are more preferable.
In the context of the present invention, the ellagitannin is geraniin.
Geraniin is characterized by molecular formula $C_{41}H_{28}O_{27}$ and has a molecular weight of 952.64, and its structural formula is expressed by the following.

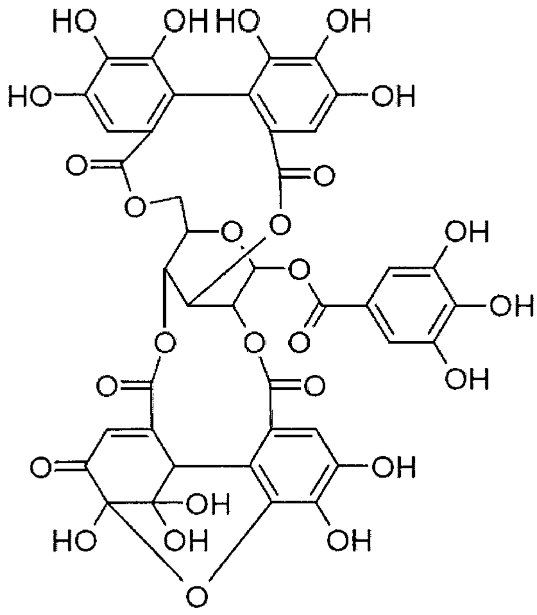

Casuarictin is characterized by molecular formula $C_{41}H_{28}O_{26}$ and has a molecular weight of 936.65, and its structural formula is characterized by the following.

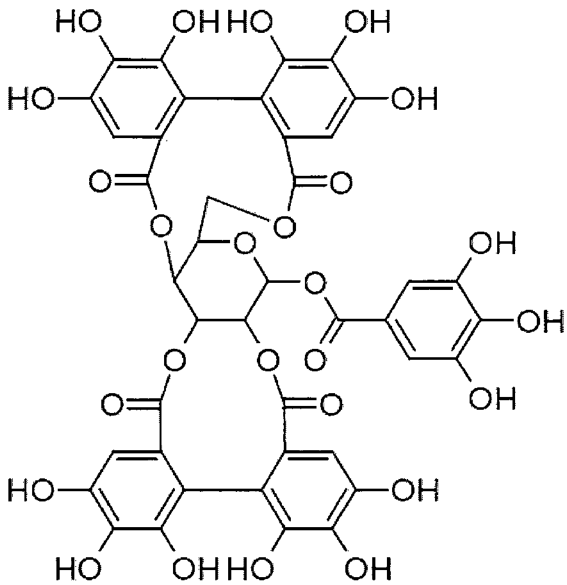

Eugeniin is characterized by molecular formula $C_{41}H_{30}O_{26}$ and has a molecular weight of 938.66, and its structural formula is characterized by the following.

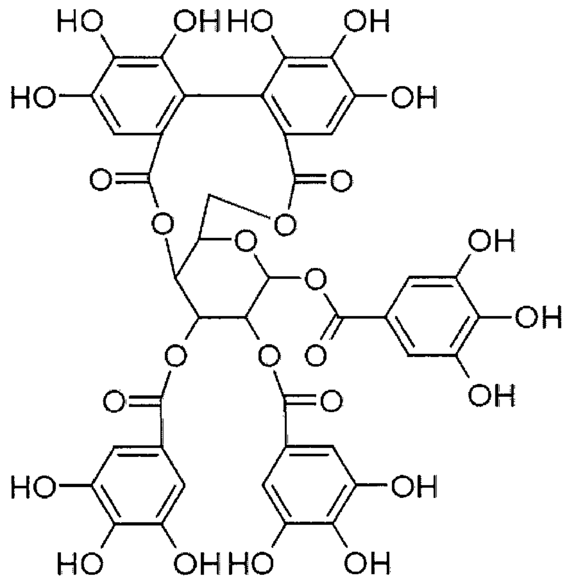

Tellimagrandin I is characterized by molecular formula $C_{34}H_{26}O_{22}$ and has a molecular weight of 786.56, and its structural formula is characterized by the following.

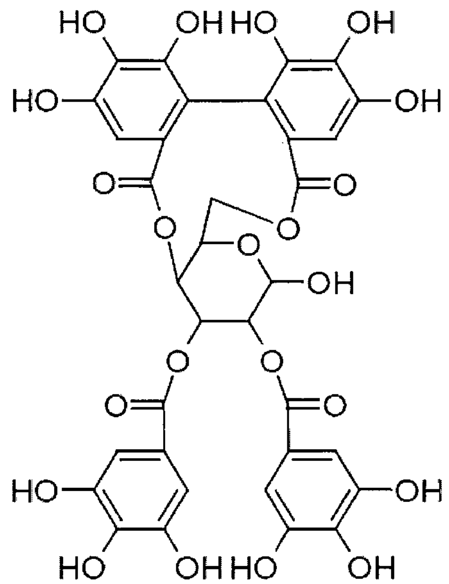

1,3-di-O-galloyl-4,6-O-hexahydroxydiphenoyl glucose is characterized by molecular formula $C_{34}H_{26}O_{22}$ and has a molecular weight of 786.56, and its structural formula is characterized by the following.

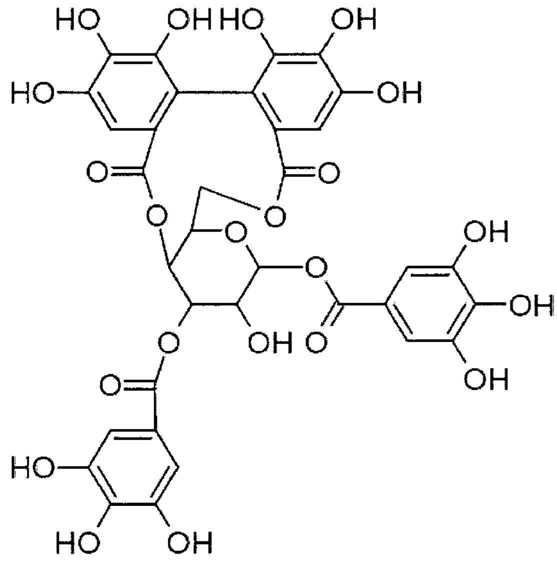

The strictinin is characterized by molecular formula $C_{27}H_{22}O_{18}$ and has a molecular weight of 634.45, and its structural formula is characterized by the following.

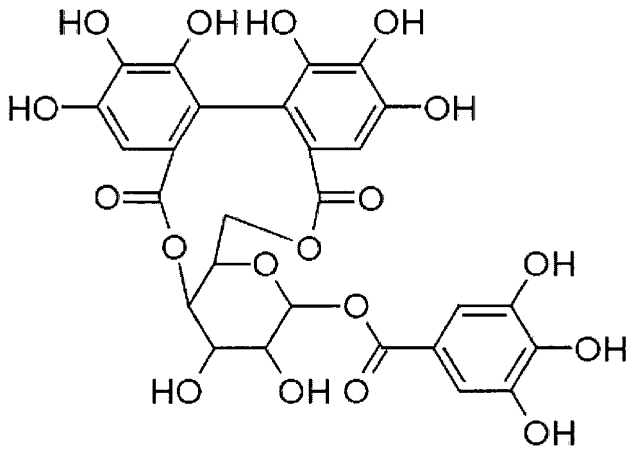

A method for confirming the structure of the ellagitannin is not particularly limited and may be various synthesis methods appropriately selected. Examples thereof include mass spectrometry, ultraviolet spectroscopy, infrared spectroscopy, proton nuclear magnetic resonance spectroscopy, and carbon-13 nuclear magnetic resonance spectroscopy.

The ellagitannin, which is, in the context of the present invention, selected from geraniin, may be in the form of salt.

The salt is not particularly limited and may be appropriately selected depending on the intended purpose. Examples thereof include acid addition salts, metal salts, ammonium salts, organic amine addition salts, and amino acid addition salts.

Specific examples of the acid addition salts include inorganic acid salts such as a hydrochloric acid salt, a sulfuric acid salt, and a phosphoric acid salt, and organic acid salts such as an acetic acid salt, a maleic acid salt, a fumaric acid salt, a tartaric acid salt, a citric acid salt, and a lactic acid salt.

Specific examples of the metal salts include alkali metal salts such as a lithium salt, a sodium salt, and a potassium salt, alkaline earth metal salts such as a magnesium salt and a calcium salt, an aluminum salt, and a zinc salt.

Specific examples of the ammonium salt include an ammonium salt and a tetramethylammonium salt.

Specific examples of the organic amine addition salts include a morpholine addition salt and a piperidine addition salt.

Specific examples of the amino acid addition salts include a glycine addition salt, a phenylalanine addition salt, an aspartic acid addition salt, a glutamic acid addition salt, and a lysine addition salt.

The ellagitannin, which is, in the context of the present invention, selected from geraniin, may be used alone, or two or more kinds thereof may be used in combination.

The ellagitannin may be a chemically synthesized product. Alternatively, the ellagitannin may be a product

purified from a plant extract containing the ellagitannin, or may be a state of being contained in the plant extract.

The plant from which the plant extract is derived is not particularly limited and may be appropriately selected depending on the intended purpose. Examples thereof include plants belonging to the genus *Thea,* plants belonging to the genus *Stachyurus,* plants belonging to the genus *Casuarina,* plants belonging to the genus *Geranium,* plants belonging to the genus *Mallotus,* plants belonging to the genus *Terminalia,* plants belonging to the genus *Sanguisorba,* plants belonging to the genus *Nuphar,* plants belonging to the genus *Hydrangea,* plants belonging to the genus *Syzygium,* plants belonging to the genus *Quercus,* plants belonging to the genus *Acer,* plants belonging to the genus *Cornus,* plants belonging to the genus *Punica,* plants belonging to the genus *Rosa,* plants belonging to the genus *Rubus,* and plants belonging to the genus *Eugenia.*

Some of the above plants are utilized as folk medicines or condiments and thus the ellagitannin can be extracted from them. Examples of the plants utilized as folk medicines or condiments include *Geranium thunbergii* belonging to the genus *Geranium, Mallotus japonicas* belonging to the genus *Mallotus,* and *Syzygium aromaticum* belonging to the genus *Eugenia,* with *Geranium thunbergii* being preferable.

The method for producing the plant extract is not particularly limited and may be appropriately selected from known methods.

For example, a to-be-extracted raw material of the plant extract is prepared by taking the fluit, the seed, the leaf, the stem, the root, the rootstock, and the like of a plant as raw materials at appropriate times. The raw material may be used directly or after being subjected to drying such as air drying.

Examples of the method for extracting ellagitannin from the extracted raw material include a method in which the to-be-extracted raw material is milled or finely cut and then extracted with an extraction solvent.

The extraction solvent is not particularly limited and may be appropriately selected depending on the intended purpose. Examples thereof include water, alcohols, ketones, esters, and acid-added water.

Specific examples of the alcohols include methanol, ethanol, and isopropyl alcohol.

Specific examples of the ketones include acetone and methyl ethyl ketone.

Specific examples of the esters include methyl acetate and ethyl acetate.

Specific examples of the acid-added water include citric acid-contaning water, acetic acid-containing water, and tartaric acid-containing water.

The extraction solvent may be used alone, or two or more kinds thereof may be used in combination.

The temperature in the extraction is not particularly limited and may be appropriately selected depending on the intended purpose. It is, for example, 0°C to 100°C.

The period of the extraction is not particularly limited and may be appropriately selected depending on the intended purpose. It is, for example, about 1 hour to about 10 days.

The amount of the extraction solvent used is not particularly limited and may be appropriately selected depending on the intended purpose. For example, it is an amount 1 time by mass to 30 times by mass the dried raw material.

The extraction operation is not particularly limited and may be appropriately selected depending on the intended purpose. For example, it is stirring and being left in a state of immersed.

The number of the extraction operations may be one time, or the extraction operations may be repeated two or more times.

The method for purifying the ellagitannin is not particularly limited and may be appropriately selected depending on the intended purpose. Examples thereof include two-phase solvent partition methods, column chromatography methods, and fractionating high performance liquid chromatography methods. These may be used alone, or two or more kinds thereof may be used in combination.

Examples of the raw material from which the ellagitanninis to be purified include an extract prepared by removing insoluble residues through filtration or centrifugation from a crude extract obtained by the above extraction operations, and squeezed liquid and sap of a plant.

Specific examples of the two-phase solvent partition methods include a method in which oil-soluble ingredients or pigments are extracted and removed with chloroform, ethyl ether, n-hexane, petroleum ether, or the like from the raw material from which the ellagitannin is to be purified, and a method in which the raw material from which the ellagitannin is to be purified is partitioned between a solvent, such as ethyl acetate, n-butanol, or methyl ethyl ketone, and water so that the ellagitannin is recovered to the solvent phase.

Specific examples of the column chromatography methods include an adsorption column chromatography method using, as a carrier, normal-phase silica gel, reverse-phase silica gel, DIAION HP-20, SEPABEADS SP-207, or the like, and a gel filtration method using SEPHADEX LH-20 or the like as a carrier.

Specific examples of the fractionating high performance liquid chromatography methods include a method using a reverse-phase column using octadecyl silica or the like, and a method using a normal-phase column using silica gel or the like.

The above purification methods remove water-soluble ionic substances, such as salts, non-ionic substances, such as saccharides and polysaccharides, oily matter, and pigments from the raw material from which the ellagitannin

is to be purified, making it possible to obtain purified ellagitannin, such as geraniin.

The amount of geraniin in the ellagitannin-containing anticancer agent is not particularly limited and may be appropriately selected depending on the intended purpose. The ellagitannin-containing anticancer agent may be geraniinitself.

<Other ingredients>

The other ingredients in the ellagitannin-containing anticancer agent are not particularly limited and may be appropriately selected depending on the intended purpose from pharmacologically acceptable carriers. Examples thereof include additives, supplements and water. These may be used alone, or two or more kinds thereof may be used in combination.

The additives or supplements are not particularly limited and may be appropriately selected depending on the intended purpose. Examples thereof include a disinfectant, a preserving agent, a binding agent, a thickener, an adhesive agent, an integrating agent, a colorant, a stabilizer, a pH adjuster, a buffer, a tonicity agent, a solvent, an antioxidant, a UV rays-preventing agent, a preventing agent for precipitation of crystals, a defoaming agent, a property improving agent and an antiseptic agent.

The disinfectant is not particularly limited and may be appropriately selected depending on the intended purpose. Examples thereof include cationic surfactants such as benzalkonium chloride, benzethonium chloride and cetylpyridinium chloride.

The preserving agent is not particularly limited and may be appropriately selected depending on the intended purpose. Examples thereof include p-hydroxybenzoate esters, chlorobutanol and clesol.

The binding agent, thickener and adhesive agent are not particularly limited and may be appropriately selected depending on the intended purpose. Examples thereof include starch, dextrin, cellulose, methyl cellulose, ethyl cellulose, carboxymethyl cellulose, hydroxyethyl cellulose, hydroxypropyol cellulose, hydroxypropyolmethyl cellulose, carboxymethyl starch, pullulan, sodium alginate, ammonium alginate, propylene glycol alginic acid esters, guar gum, locust bean gum, gum Arabic, xanthane gum, gelatin, casein, polyvinyl alcohol, polyethylene oxide, polyethylene glycol, ethylene/propylene block polymers, sodium polyacrylates and polyvinylpyrrolidone.

The integrating agent is not particularly limited and may be appropriately selected depending on the intended purpose. Examples of the integrating agent include water, ethanol, propanol, simple syrup, glucose liquid, starch liquid, gelatin liquid, carboxymethyl cellulose, hydroxypropyl cellulose, hydroxypropyl starch, methyl cellulose, ethyl cellulose, shellac, calcium phosphate and polyvinylpyrrolidone.

The colorant is not particularly limited and may be appropriately selected depending on the intended purpose. Examples thereof include titanium oxide and iron oxide.

The stabilizer is not particularly limited and may be appropriately selected depending on the intended purpose. Examples thereof include tragacanth, gum Arabic, gelatin, sodium pyrosulfite, ethylenediaminetetraacetate (EDTA), thioglycolic acid and thiolactic acid.

The pH adjuster or the buffer is not particularly limited and may be appropriately selected depending on the intended purpose. Examples thereof include sodium citrate, sodium acetate and sodium phosphate.

The tonicity agent is not particularly limited and may be appropriately selected depending on the intended purpose. Examples thereof include sodium chloride and glucose.

The amount of the other ingredients in the ellagitannin-containing anticancer agent is not particularly limited and may be appropriately selected depending on the intended purpose so long as the effects of the present invention are not impeded.

The ellagitannin-containing anticancer agent may be used alone or in combination with drugs or medicaments containing other ingredients as active ingredients. Also, the ellagitannin-containing anticancer agent may be used in a state of being formulated into drugs or medicaments containing other ingredients as active ingredients.

The method for examining whether the ellagitannin has anticancer effects is not particularly limited and may be appropriately selected from known methods. Examples thereof include a method described in Test Example 2 described below.

<Dosage form>

The dosage form of the ellagitannin-containing anticancer agent is not particularly limited and may be appropriately selected depending on the intended purpose. Examples thereof include a solid preparation, a semi-solid preparation and a liquid preparation.

-Solid preparation-

The solid preparation is not particularly limited and may be appropriately selected depending on the intended purpose. When it is used as an internal preparation, examples of the solid preparation include tablets, chewable tablets, foaming tablets, orally-disintegrating tablets, troches, drops, hard capsules, soft capsules, granules, powder, pills, dry syrups and infusions.

When the solid preparation is an external preparation, examples of the solid preparation include suppositories, cataplasms and plasters.

-Semi-solid preparation-

The semi-solid preparation is not particularly limited and may be appropriately selected depending on the intended purpose. When it is used as an internal preparation, examples of the semi-solid preparation include electuaries, chewing gums, whip and jelly.

When the semi-solid preparation is used as an external preparation, examples of the semi-solid preparation include ointments, cream, mousse, inhaler and nasal gel.

-Liquid preparation-

The liquid preparation is not particularly limited and may be appropriately selected depending on the intended purpose. When it is used as an internal preparation, examples of the liquid preparation include syrups, drinks, suspensions and spirits.

When the liquid preparation is used as an external preparation, examples of the liquid preparation include liquid, eye drops, aerosol and sprays.

<Administration>

The administration method, administration dose, administration period and administration target of the ellagitannin-containing anticancer agent are not particularly limited and may be appropriately selected depending on the intended purpose.

Examples of the administration method include a local administration method, an enteral administration method and a parenteral administration method.

The administration dose is not particularly limited and may be appropriately selected considering various factors of an administration target, such as the age, body weight, constitution, symptom and the presence or absence of administration of drugs or medicaments containing other ingredients as active ingredients.

The animal species serving as the administration target is not particularly limited and may be appropriately selected depending on the intended purpose. Examples thereof include human, monkey, pig, bovine, sheep, goat, dog, cat, mouse, rat and bird. Among them, it can be suitably used for human.

The ellagitannin-containing anticancer agent has excellent anticancer effects and thus can be suitably used as a preventing agent or a therapeutic agent for cancers. The anticancer agent can be used suitably as a preventing agent or a therapeutic agent for osteosarcoma and lung cancer among cancers.

(Combination use anticancer agent)

A combination use anticancer agent of the present invention contains at least the ellagitannin-containing anticancer agent which is geraniin, and an anticancer agent containing anthracyclines (hereinafter may be referred to as "anthracyclines-containing anticancer agent"), which is in the context of the present invention, doxorubicin; and, if necessary, further contains other ingredients.

<Ellagitannin-containing anticancer agent>

Details of the ellagitannin-containing anticancer agent are as described at the section of the anticancer agent of the present invention described above.

<Anthracyclines-containing anticancer agent>

The anthracyclines-containing anticancer agent contains at least doxorubicin, and, if necessary, further contains other ingredients.

- Anthracyclines-

The anthracyclines are glycosides having 7,8,9,10-tetrahydro-5,12-tetracenequinone as a mother nucleus thereof and include substances known to have antitumor effects against various cancers.
The anthracyclines are generally not particularly limited and may be appropriately selected depending on the intended purpose. They are preferably doxorubicin, daunorubicin, epirubicin, idarubicin, valrubicin, and mitoxantrone, and, in the context of the present invention, are selected from doxorubicin. The anthracyclines, which are, in the context of the present invention, selected from doxorubicin,may be used alone, or two or more kinds thereof may be used in combination.
The anthracyclines, which are, in the context of the present invention, selected from doxorubicin, may be in the form of salt.
The salt is not particularly limited and may be appropriately selected depending on the intended purpose. Examples thereof include a hydrochloric acid salt.
The anthracyclines may be a chemically synthesized product or a product purified from a culture of bacteria. Alternatively, the anthracyclines may be a commercially available product.
The amount of the anthracyclines, and, specifically, doxorubicin in the context of the invention, in the anthracyclines-containing anticancer agent is not particularly limited and may be appropriately selected depending on the intended purpose. The anthracyclines-containing anticancer agent may be doxorubicin itself.

-Other ingredients-

The other ingredients in the anthracyclines-containing anticancer agent are not particularly limited and may be appropriately selected depending on the intended purpose from pharmacologically acceptable carriers. Examples thereof include additives, supplements and water. These may be used alone, or two or more kinds thereof may be used in combination.
Examples of the additives or supplements include additives or supplements similar to those described at the section of the other ingredients of the ellagitannin-containing anticancer agent described above.
The amount of the other ingredients in the anthracyclines-containing anticancer agent is not particularly limited and may be appropriately selected depending on the intended purpose so long as the effects of the present invention are not impeded.
The combination use anticancer agent may use only the ellagitannin-containing anticancer agent, which, in the context of the present invention, contains geraniin, and the anthracyclines-containing anticancer agent, which, in the context of the present invention, contains doxorubicin, in combination, or may use them in combination with drugs or medicaments containing other ingredients as active ingredients. Alternatively, the combination use anticancer agent may be used in a state of being formulated into drugs or medicaments containing other ingredients as active ingredients.

<Dosage form>

A dosage form of the combination use anticancer agent is not particularly limited and may be appropriately selected depending on the intended purpose. Examples thereof include dosage forms similar to those described at the section of the dosage form of the ellagitannin-containing anticancer agent described above.
The ellagitannin-containing anticancer agent and the anthracyclines-containing anticancer agent may have the same dosage form or different dosage forms.

<Administration>

The administration method, administration dose, administration period and administration target of the combination use anticancer agent are not particularly limited and may be appropriately selected depending on the intended purpose. Examples thereof include administration methods, administration doses, administration periods and administration targets similar to those described at the section of the dosage form of the ellagitannin-containing anticancer agent described above.
In the combination use anticancer agent, the ellagitannin-containing anticancer agent, which, in the context of the present invention, comprises geraniin,and the anthracyclines-containing anticancer agent, which, in the context of the present invention, comprises doxorubicin, may be administered in separate dosage forms, or may be administered as a single agent containing both of them in one drug product.
When said ellagitannin-containing anticancer agent and said anthracyclines-containing anticancer agent are formed into separate dosage forms, both of them may be administered simultaneously or may be separately with a time interval being provided.

The order of said ellagitannin-containing anticancer agent and said anthracyclines-containing anticancer agent to be administered when they are administered separately with a time interval being provided is not particularly limited and may be appropriately selected depending on the intended purpose.

Administration routes of said ellagitannin-containing anticancer agent and said anthracyclines-containing anticancer agent may be the same or different.

The combination use anticancer agent has excellent anticancer effects and thus can be suitably used as a preventing agent or a therapeutic agent for cancers. The combination use anticancer agent can be used suitably as a preventing agent or a therapeutic agent for osteosarcoma and lung cancer among cancers.

(Method for preventing or treating cancer)

When administered to individuals suffering from cancer, the ellagitannin-containing anticancer agent, which, according to the invention, comprises geraniin, and the combination use anticancer agent can effectively suppress proliferation of cancer cells and prevent or treat cancer. Accordingly, said agents may be used in a preventing or treating method for cancer, which includes administering, to an individual, the ellagitannin-containing anticancer agent, the combination use anticancer agent, or both thereof.

The cancer to be targeted is not particularly limited and may be appropriately selected depending on the intended purpose. It can be suitably used for osteosarcoma and lung cancer.

Examples

The present invention will next be described by way of Test Examples.

(Test Example 1: *in vitro* cell proliferation suppressive activity)

Proliferation suppressive activities of ellagitannin against various kinds of cancer cell lines were measured in the following manner.

Each of the following cancer cell lines was placed in a 96-well plate at $5 \times 10^3$ cell/100 μL per well using a DMEM medium (product of NISSUI PHARMACEUTICAL CO., LTD.) containing FBS (product of Sigma-Aldrich Co.) in an amount of 10 liquid amount %, and was cultured for 24 hours at 37°C in an atomosphere of 5%$CO_2$, whereby the cells were allowed to adhere to the plate completely.

Each of the following ellagitannins was added thereto so as to have final concentrations of 100 μg/mL, 50 μg/mL, 25 μg/mL, 12.5 μg/mL, 6.25 μg/mL, 3.13 μg/mL, 1.56 μg/mL, and 0.78 μg/mL, followed by culturing for 3 days at 37°C in an atomosphere of 5%$CO_2$.

In addition, as comparative controls, the 96-well plates to which the cancer cells had been allowed to adhere were cultured for 3 days at 37°C in an atomosphere of 5%$CO_2$ without addition of ellagitannin.

After the culturing for 3 days, cell proliferation was measured using the MTT method. The MTT method was performed as follows. Specifically, 10 μL of an MTT solution (a PBS solution containing MTT (3-(4,5-dimethylthiazol-2-yl)-2,5-diphenyltetrazolium bromide) at a concentration of 5 mg/mL) was added to each well and cultured for 4 hours. The formed formazan products were dissolved by adding 100 μL of a 20%SDS solution to each well, and measured for absorbance at 570 nm using LABSYSTEMS MULTISCAN MS (product of DS Pharma Biomedical Co., Ltd.).

The absorbance of the system involving the addition of the ellagitannin was indicated as "A" and the absorbance of the system of the comparative controls was indicated as "B", and cell proliferation inhibitory rate (%) was calculated from the following formula (1).

$$\text{Cell proliferation inhibitory rate (\%)} = \{(B-A)/B\}\times 100 \ldots \text{Formula (1)}$$

Next, the cell proliferation inhibitory rate was used to determine a concentration at which 50% of the cell proliferation was suppressed ($IC_{50}$ value). The results are presented in Table 1.

<Cancer cell lines>

- Human osteosarcoma SJSA-1 cells (obtained from ATCC)
- Human lung cancer NCI-H460 cells (obtained from ATCC)

<Ellagitannin>

- Geraniin (product of Chengdu Biopurify Phytochemicals Co.)
- Casuarictin (product of Nagara Science Co., Ltd.)
- Eugeniin (product of Nagara Science Co., Ltd.)
- Tellimagrandin I (product of Nagara Science Co., Ltd.)
- 1,3-Di-O-galloyl-4,6-O-hexahydroxydiphenoyl glucose (product of Nagara Science Co., Ltd.)
- Strictinin (product of Nagara Science Co., Ltd.)

Table 1

| Ellagitannins | $IC_{50}$ (μg/mL) | |
|---|---|---|
| | SJSA-1 cells | NCI-H460 cells |
| Geraniin | 100 | 40 |
| Casuarictin | 61 | 35 |
| Eugeniin | 18 | 5.6 |
| Tellimagrandin I | 46 | 32 |
| 1,3-Di-O-galloyl-4,6-O-hexahydroxydiphenoyl glucose | 41 | 11 |
| Strictinin | 110 | 33 |

It has been found from the results of Table 1 that the ellagitannins have activity of suppressing proliferation of various cancer cells *in vitro.*

(Test Example 2-1: Antitumor activity-1 in cancer cell-transplanted mice)

Geraniin (product of Chengdu Biopurify Phytochemicals Co.) was used as the ellagitannin and was studied for antitumor activity in the following manner.

Each of the following cancer cell lines was prepared so as to be $1 \times 10^7$ cells/100 μL in 50% by volume MATRIGEL (product of Becton, Dickinson and Company) containing a DMEM medium, and 100 μL of the resultant product was subcutaneously transplanted into a female BALB/c/nu/nu mouse (nude mouse) having a body weight of about 18 g.

From one week after the transplantation, the geraniin was administered to the tail vein of the mouse twice a week (every 3 or 4 days). The geraniin administered was prepared by dissolving the geraniin in a physiological saline containing 5% DMSO and 0.5% Tween80 so that the geraniin became 12.5 mg/kg as the liquid amount to be administered.

In addition, as comparative controls, from one week after the transplantation, a physiological saline (Saline) containing 5% DMSO and 0.5% Tween80 was administered to the tail vein of the mouse twice a week (every 3 or 4 days).

Note that, the number of the mice used for the test was 10 per group in the test using human osteosarcoma SJSA-1 cells as the cancer cell line, and was 5 per group in the test using human lung cancer NCI-H460 cells as the cancer cell line.

The antitumor effect of the geraniin was evaluated by observing the proliferation state of the tumor after the transplantation of the cells. Specifically, a longer diameter and a shorter diameter of each tumor were meatured with a caliper, and a tumor volume was measured from the following formula (2). Its average and standard deviation were obtained to determine the effect in the geraniin-administration groups in comparison to the comparative control groups. The results are presented in Figs. 1 and 2.

$$\text{Tumore volume} = \text{longer diameter} \times (\text{shorter diameter})^2 \div 2 \ldots \text{Formula (2)}$$

<Cancer cell lines>

- Human osteosarcoma SJSA-1 cells (obtained from ATCC)

- Human lung cancer NCI-H460 cells (obtained from ATCC)

Fig. 1 indicates a change of tumor volume in mice transplanted with human osteosarcoma SJSA-1 cells, and Fig. 2 indicates a change of tumor volume in mice transplanted with human lung cancer NCI-H460 cells.

In Figs. 1 and 2, "*" means a significant difference of 0.05% or less, "**" means a significant difference of 0.01% or less, "***" means a significant difference of 0.001% or less, "black circle" indicates the results of the group to which a physiological saline was administered (Saline), and "white circle" indicates the results of the geraniin-administration groups.

It has been found from the results of Figs. 1 and 2 that the geraniin-administration groups were significantly smaller than the comparative control groups in terms of the tumor volume in the mice transplanted with human osteosarcoma SJSA-1 cells and the tumor volume in the mice transplanted with human lung cancer NCI-H460 cells, indicating that geraniin which is ellagitannin has anticancer effects.

(Comparative Example 2-2: Antitumor activity-2 in cancer cell-transplanted mice)

Eugeniin (product of Nagara Science Co., Ltd.) was used as the ellagitannin and was studied for antitumor activity in the following manner.

Cancer cell line, human osteosarcoma SJSA-1 cells (obtained from ATCC) were prepared so as to be $1 \times 10^7$ cells/100 μL in 50% by volume MATRIGEL (product of Becton, Dickinson and Company) containing a DMEM medium, and 100 μL of the resultant product was subcutaneously transplanted into a female BALB/c/nu/nu mouse (nude mouse) having a body weight of about 18 g.

From one week after the transplantation, the eugeniin was administered to the tail vein of the mouse twice a week (every 3 or 4 days). The eugeniin administered was prepared by dissolving the eugeniin in a physiological saline containing 5% DMSO and 0.5% Tween80 so that the eugeniin became 12.5 mg/kg, 6.25 mg/kg, or 3.13 mg/kg as the liquid amount to be administered.

In addition, as comparative controls, from one week after the transplantation, a physiological saline (Saline) containing 5% DMSO and 0.5% Tween80 was administered to the tail vein of the mouse twice a week (every 3 or 4 days).

Note that, the number of the mice used for the test was 7 per group.

The antitumor effect of the eugeniin was evaluated in the same manner as in Test Example 2-1. The results are presented in Fig. 3.

Fig. 3 indicates a change of tumor volume in mice transplanted with human osteosarcoma SJSA-1 cells.

In Fig. 3, "*" means a significant difference of 0.05% or less, "**" means a significant difference of 0.01% or less, "black circle" indicates the results of the group to which a physiological saline was administered (Saline), "white circle" indicates the results of the group to which eugeniin was administered at 12.5 mg/kg, "triangle" indicates the results of the group to which eugeniin was administered at 6.25 mg/kg, and "square" indicates the results of the group to which eugeniin was administered at 3.13 mg/kg.

It has been found from the results of Fig. 3 that the eugeniin-administration groups were significantly smaller than the comparative control groups in terms of the tumor volume in the mice transplanted with human osteosarcoma SJSA-1 cells, indicating that eugeniin which is ellagitannin has anticancer effects.

(Test Example 2-3: Antitumor activity-3 in cancer cell-transplanted mice)

A *Geranium thunbergii* extract containing geraniin prepared in the following manner was used as the ellagitannin and was studied for antitumor activity in the following manner.

<Preparation of *Geranium thunbergii* extract>

Ethanol (3 L) was added to 500 g of *Geranium thunbergii* (product of Tochimoto Tenkaido Co., Ltd.). The resultant mixture was left to stand still for 24 hours, followed by filtration. The filtrate was dried to be solidified, and was used as the *Geranium thunbergii* extract.

-Measurement of geraniin content of the *Geranium thunbergii* extract-

The *Geranium thunbergii* extract was analyzed through HPLC.

Column: Nucrosil C18 (diameter: 4.6 mm × 250 mm) (product of GL Sciences Inc.)
Detection wavelength: 220 nm
Flow rate: 0.5 mL/min
Mobile phase liquid A: 0.05% trifluoroacetic acid (TFA)

Mobile phase liquid B: 100% acetonitrile
Gradient conditions: (liquid A 100%, liquid B 0%, 0 min)→(liquid A 70%, liquid B 30%, 50 min)→(liquid A 0%, liquid B 100%, 70 min)

A peak area of geraniin analyzed through the HPLC was indicated by "A" and a total value of all the peak areas was indicated by "B", and the geraniin content (% by mass) was calculated from the following formula (3). As a result, the *Geranium thunbergii* extract was found to contain geraniin in an amount of 8.5% by mass.

$$\text{Geraniin content (\% by mass)} = A/B \times 100 \ldots \text{Formula (3)}$$

<Measurement of antitumor activity>

Cancer cell line, human osteosarcoma SJSA-1 cells (obtained from ATCC) were prepared so as to be $5 \times 10^6$ cells/100 μL in 50% by volume MATRIGEL (product of Becton, Dickinson and Company) containing a DMEM medium, and 100 μL of the resultant product was subcutaneously transplanted into a female BALB/c/nu/nu mouse (nude mouse) having a body weight of about 18 g.

From one week after the transplantation, the *Geranium thunbergii* extract was administered to the tail vein of the mouse twice a week (every 3 or 4 days). The *Geranium thunbergii* extract administered was prepared by dissolving the *Geranium thunbergii* extract in a physiological saline containing 5% DMSO and 0.5% Tween80 so that the *Geranium thunbergii* extract became 125 mg/kg (geraniin was 10.6 mg/kg), 62.5 mg/kg (geraniin was 5.3 mg/kg), or 31.3 mg/kg (geraniin was 2.7 mg/kg) as the liquid amount to be administered.

In addition, as comparative controls, from one week after the transplantation, a physiological saline (Saline) containing 5% DMSO and 0.5% Tween80 was administered to the tail vein of the mouse twice a week (every 3 or 4 days).

Note that, the number of the mice used for the test was 7 per group.

The antitumor effect of the *Geranium thunbergii* extract was evaluated in the same manner as in Test Example 2-1. The results are presented in Fig. 4.

Fig. 4 indicates a change of tumor volume in mice transplanted with human osteosarcoma SJSA-1 cells.

In Fig. 4, "**" means a significant difference of 0.01% or less, "***" means a significant difference of 0.001% or less, "black circle" indicates the results of the group to which a physiological saline was administered (Saline), "white circle" indicates the results of the group to which the *Geranium thunbergii* extract was administered at 125 mg/kg, "triangle" indicates the results of the group to which the *Geranium thunbergii* extract was administered at 62.5 mg/kg, and "square" indicates the results of the group to which the *Geranium thunbergii* extract was administered at 31.3 mg/kg.

It has been found from the results of Fig. 4 that the *Geranium thunbergii* extract-administration groups were significantly smaller than the comparative control groups in terms of the tumor volume in the mice transplanted with human osteosarcoma SJSA-1 cells, indicating that the *Geranium thunbergii* extract containing geraniin which is ellagitannin has anticancer effects.

It has been indicated from the results of Test Examples 2-1 to 2-3 that the ellagitannin can be used in an anticancer agent.

(Test Example 3: Antitumor activity-4 in cancer cell-transplanted mice)

Antitumor activity when ellagitannin and an anthracyclines-containing anticancer agent, which is an existing anticancer agent, were used in combination was studied in the following manner.

Geraniin (product of Chengdu Biopurify Phytochemicals Co.) was used as the ellagitannin. A doxorubicin hydrochloride solution (product of Kyowa Hakko Kirin Co., Ltd.) was used as the anthracyclines-containing anticancer agent.

Cancer cell line, human osteosarcoma SJSA-1 cells (obtained from ATCC) were prepared so as to be $5 \times 10^6$ cells/100 μL in 50% by volume MATRIGEL (product of Becton, Dickinson and Company) containing a DMEM medium, and 100 μL of the resultant product was subcutaneously transplanted into a female BALB/c/nu/nu mouse (nude mouse) having a body weight of about 18 g.

From the following day of the transplantation, (1) geraniin, (2) doxorubicin hydrochloride solution, or (3) mixed solution of geraniin and doxorubicin hydrochloride solution was administered to the tail vein of the mouse twice a week (every 3 or 4 days).

The (1) geraniin was prepared by dissolving the geraniin in a physiological saline containing 5% DMSO and 0.5% Tween80 so that the geraniin became 12.5 mg/kg as the liquid amount to be administered. The (2) doxorubicin hydrochloride solution was prepared by dissolving doxorubicin hydrochloride in a physiological saline so that the doxorubicin hydrochloride became 2.5 mg/kg as the liquid amount to be administered. The (3) mixed solution of geraniin and

doxorubicin hydrochloride solution was prepared by mixing each solution so that the geraniin became 12.5 mg/kg as the liquid amount to be administered and the doxorubicin hydrochloride became 2.5 mg/kg as the liquid amount to be administered.

In addition, as comparative controls, from one week after the transplantation, a physiological saline (Saline) containing 5% DMSO and 0.5% Tween80 was administered to the tail vein of the mouse twice a week (every 3 or 4 days).

Note that, the number of the mice used for the test was 5 per group.

The antitumor effects of the (1) geraniin, the (2) doxorubicin hydrochloride solution, or the (3) mixed solution of geraniin and doxorubicin hydrochloride solution were evaluated in the same manner as in Test Example 2-1. The results are presented in Fig. 5.

Fig. 5 indicates a change of tumor volume in mice transplanted with human osteosarcoma SJSA-1 cells.

In Fig. 5, "*" means a significant difference of 0.05% or less, "**" means a significant difference of 0.01% or less, "***" means a significant difference of 0.001% or less, "black circle" indicates the results of the group to which a physiological saline was administered (Saline), "square" indicates the results of the group to which the (1) geraniin was administered (Geraniin 12.5 mg/kg), "triangle" indicates the results of the group to which the (2) doxorubicin hydrochloride solution was administered (Doxorubicin 2.5 mg/kg), and "rhombus" indicates the results of the group to which the (3) mixed solution of geraniin and doxorubicin hydrochloride solution was administered (Geraniin 12.5 mg/kg + Doxorubicin 2.5 mg/kg).

It has been found from the results of Fig. 5 that the group to which the (3) mixed solution of geraniin and doxorubicin hydrochloride solution was administered was significantly smaller than the group to which the (1) geraniin was administered and the group to which the (2) doxorubicin hydrochloride solution in terms of the tumor volume in the mice transplanted with human osteosarcoma SJSA-1 cells, indicating that remarkable antitumor actibity was exhibited by using in combination the geraniin, which is the ellagitannin, and the doxorubicin hydrochloride, which is the anthracyclines-containing anticancer agent.

Industrial Applicability

The anticancer agent and combination use anticancer agent of the present invention have excellent anticancer effects and thus can be suitably used as preventive agents or therapeutic agents for cancers, preferably as preventive agents or therapeutic agents for osteosarcoma and lung cancer.

## Claims

1. An anticancer agent for use in preventing or treating osteosarcoma or lung cancer, wherein the anticancer agent comprises geraniin.

2. A combination use anticancer agent,
comprising geraniin and doxorubicin.

## Patentansprüche

1. Ein Antikrebs-Mittel zur Verwendung zur Vorbeugung oder Behandlung von Osteosarkom oder Lungenkrebs, wobei das Antikrebs-Mittel Geraniin umfasst.

2. Ein Antikrebs-Mittel zur Kombinationsverwendung, umfassend Geraniin und Doxorubicin.

## Revendications

1. Agent anticancéreux pour une utilisation dans la prévention ou le traitement de l’ostéosarcome ou du cancer du poumon, dans lequel l’agent anticancéreux comprend de la géraniine.

2. Agent anticancéreux pour une utilisation en combinaison, comprenant de la géraniine et de la doxorubicine.

FIG. 1

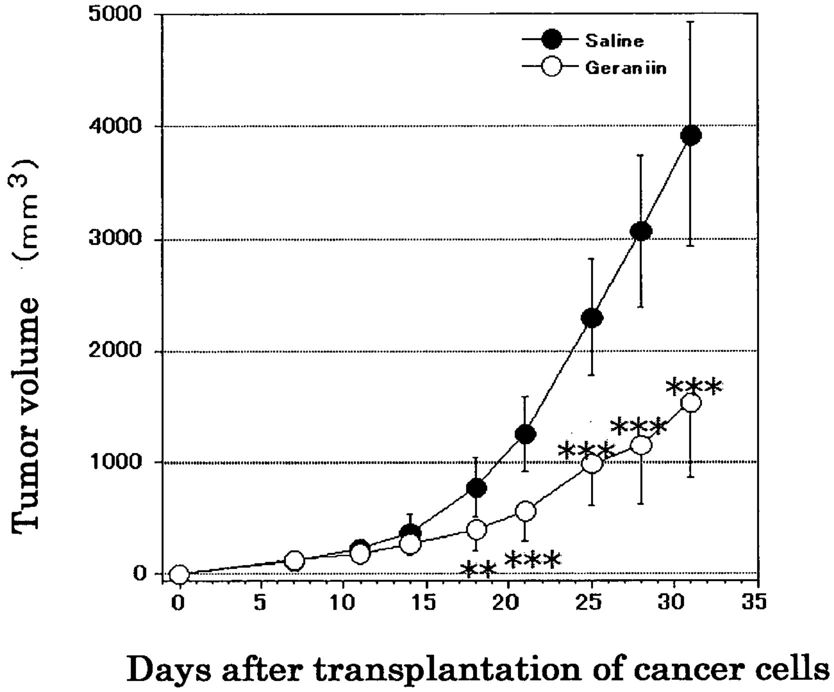
5000
4000
3000
2000
1000
0
Tumor volume (mm$^3$)
Saline
Geraniin
**
***
***
***
***
0
5
10
15
20
25
30
35
Days after transplantation of cancer cells

FIG. 2

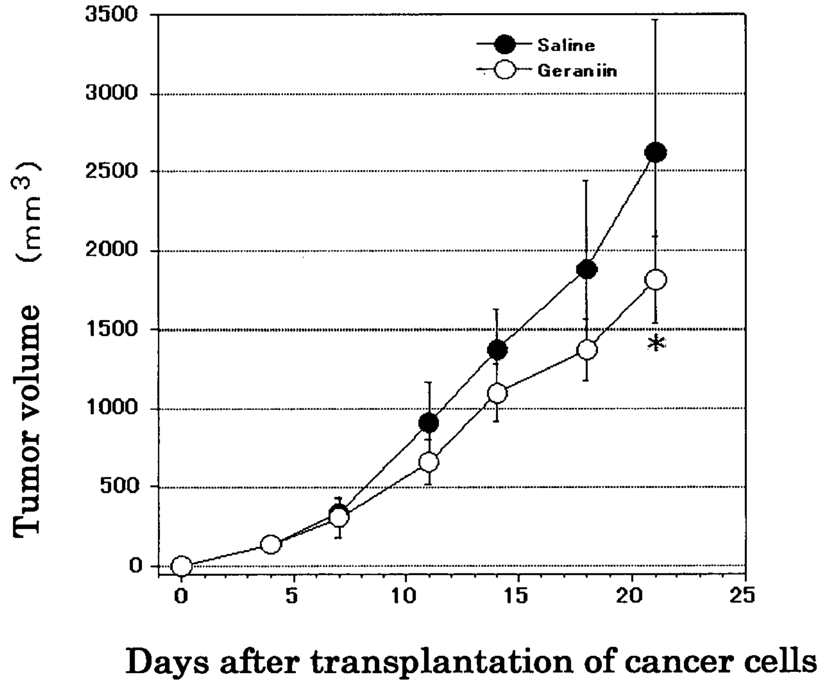
3500
3000
2500
2000
1500
1000
500
0
Tumor volume (mm$^3$)
Saline
Geraniin
*
0
5
10
15
20
25
Days after transplantation of cancer cells

FIG. 3

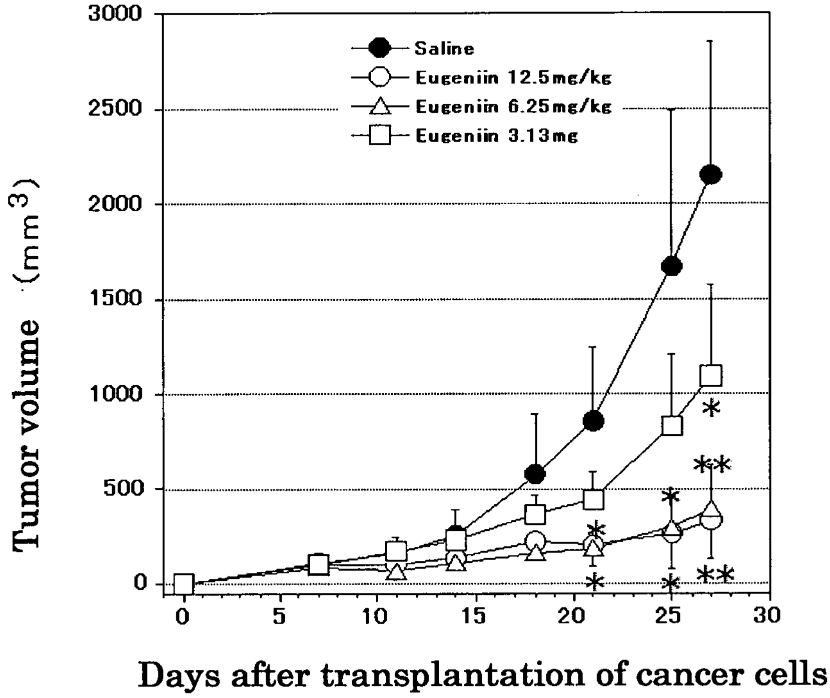
Saline
Eugeniin 12.5mg/kg
Eugeniin 6.25mg/kg
Eugeniin 3.13mg
Tumor volume (mm$^3$)
3000
2500
2000
1500
1000
500
0
0
5
10
15
20
25
30
Days after transplantation of cancer cells

FIG. 4

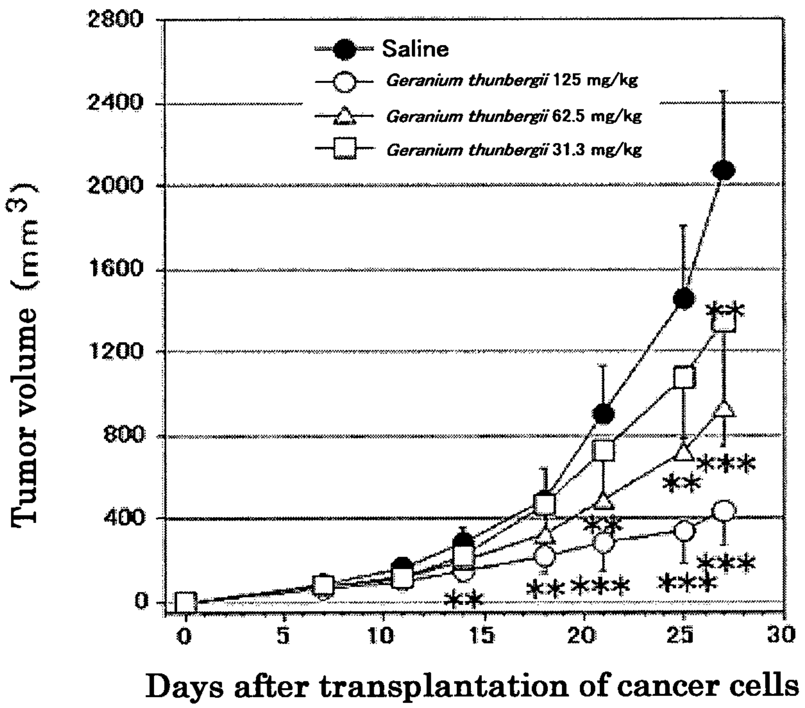
Saline
*Geranium thunbergii* 125 mg/kg
*Geranium thunbergii* 62.5 mg/kg
*Geranium thunbergii* 31.3 mg/kg
Tumor volume (mm$^3$)
2800
2400
2000
1600
1200
800
400
0
0
5
10
15
20
25
30
Days after transplantation of cancer cells

FIG. 5

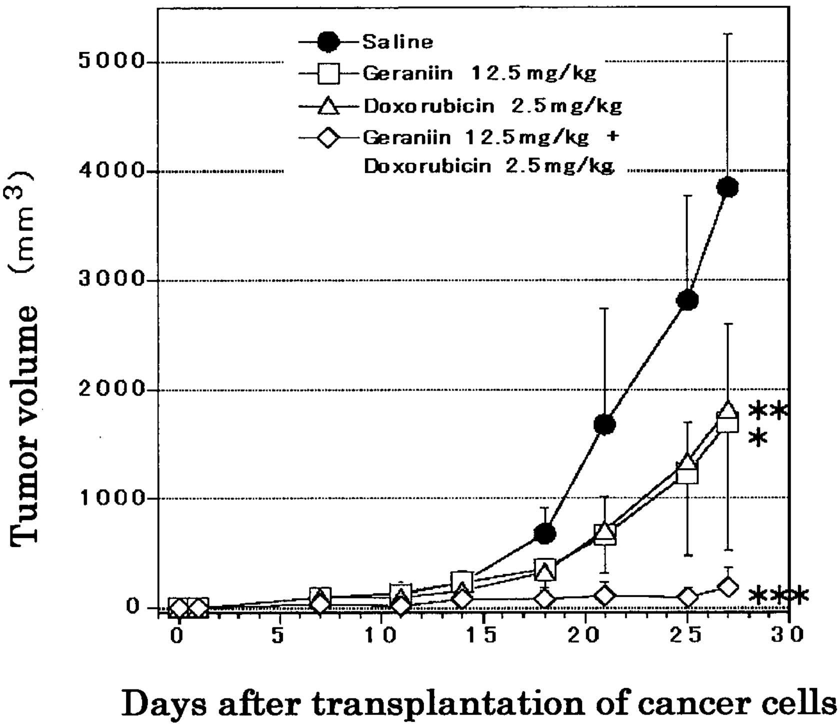
Saline
Geraniin 12.5 mg/kg
Doxorubicin 2.5mg/kg
Geraniin 12.5mg/kg + Doxorubicin 2.5mg/kg
5000
4000
3000
2000
1000
0
Tumor volume (mm3)
0
5
10
15
20
25
30
**
*
***
Days after transplantation of cancer cells

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4036239 A
- JP 6183958 A
- JP 2004091390 A


### Non-patent literature cited in the description


- **LEE et al.** *Mol Nutr Food Res.,* June 2008, vol. 52 (6), 655-63